# EUROPEAN PATENT SPECIFICATION

(11) **EP 2 020 244 B1**
(45) Date of publication and mention of the grant of the patent: **05.03.2014**
(21) Application number: 07743284.7
(22) Date of filing: 14.05.2007
(51) Int. Cl.: A61K 49/00, A61B 5/055, G01R 33/28

(54) **Magnetic resonance contrast medium using polyethylene glycol and magnetic resonance image pick-up method**
Magnetresonanz-Kontrastmittel mit Polyethylenglykol und Magnetresonanz-Bild-Aufnahmeverfahren
Agent de contrast pour résonance magnétique contenant du polyéthylène-glycol et procédé d'acquisition d'images par résonance magnétique

(30) Priority: 17.05.2006 JP 2006138236
(43) Date of publication of application: 04.02.2009
(73) Proprietor: Otsuka Pharmaceutical Co., Ltd., Tokyo 101-8585 (JP)
(72) Inventor: SUZUKI, Yoshikazu, Tokushima-shi, Tokushima 771-0182 (JP); MIURA, Iwao, Tokushima-shi, Tokushima 771-0182 (JP); IIDA, Mitsuru, Tokushima-shi, Tokushima 771-0182 (JP)
(74) Representative: HOFFMANN EITLE
(86) International application number: PCT/JP2007/059849
(87) International publication number: WO 2007/132806

(56) References cited:
- WO-A1-96/07670
- WO-A1-98/57578
- US-B1- 6 210 655
- HAROLD C. JARRELL ET AL: "13 C- 13 C Correlations and Internuclear Distance Measurements with 2D-MELODRAMA", JOURNAL OF THE AMERICAN CHEMICAL SOCIETY, vol. 120, no. 40, 1 October 1998 (1998-10-01), pages 10453-10462, XP55044357, ISSN: 0002-7863, DOI: 10.1021/ja980605j
- KERSTIN RIEDEL ET AL: "TEDOR with adiabatic inversion pulses: Resonance assignments of 13C/15N labelled RNAs", JOURNAL OF BIOMOLECULAR NMR, KLUWER ACADEMIC PUBLISHERS, DO, vol. 31, no. 1, 1 January 2005 (2005-01-01), pages 49-57, XP019249498, ISSN: 1573-5001
- DATABASE BIOSIS [Online] BIOSCIENCES INFORMATION SERVICE, PHILADELPHIA, PA, US 1987 BALLARDINI G ET AL: 'COLLAGEN AND BASEMENT MEMBRANES DISTRIBUTION IN DIMETHYLNITROSAMINE DMN INDUCED RAT LIVER CIRRHOSIS RESEMBLE HUMAN PATHOLOGY PATTERNS' Database accession no. PREV198834029189 & JOURNAL OF HEPATOLOGY, vol. 5, no. SUPPL. 1, 1987, page S86, 22ND MEETING OF THE EUROPEAN ASSOCIATION FOR THE STUDY OF THE LIVER, TORINO, ITALY, SEPTEMBER 3-5, 1 ISSN: 0168-8278

## Description

### TECHNICAL FIELD

The present invention relates to a magnesium resonance imaging method employing magnetic resonance contrast agents using polyethylene glycol, i.e., magnetic resonance contrast agents to continuously acquire magnetic resonance signals by applying excitation pulses with a repetition time of 60 seconds or less (preferably 1 second or less, more preferably 250 milliseconds or less, and particularly preferably 100 milliseconds or less). The invention also relates to a method for acquiring magnetic resonance signals, using the magnetic resonance contrast agent.

### BACKGROUND ART

In recent diagnostic imaging that utilizes contrast agents, imaging techniques using positrons or radioactively labeled contrast agents (such as PET, SPECT, and the like) and MRI (magnetic resonance imaging) that utilizes nuclear magnetic resonance have been in practical use. Although it is capable of obtaining quantitative information on a lesion using PET or SPECT, these techniques are disadvantageous in that the contrast agents cannot be stably stored because the radioactivities of the contrast agents decay with their half-life. These techniques are also not desirable for subjects because the radioactive compounds may have an adverse effect on the human body. On the other hand, MRI, which measures stable isotope nuclei, is an imaging technique that is safe for the human body, and can also advantageously obviate the problematic radioisotopes instability. For these reasons, the use of MRI is expected to expand even further.

MRI has typically employed 1H as the target nuclei of nuclear magnetic resonance, and known contrast agents therefor include Gd contrast agents, which are gadolinium (Gd) coordination compounds, colloid preparations of superparamagnetic iron oxide (SPIO) using iron oxide particles, and the like. These contrast agents utilize the principle that the relaxation time of 1H of water molecule present in a subject is shortened to thereby indirectly visualize the presence of 1H. However, MRI that utilizes 1H as the target nuclei of nuclear magnetic resonance does not have a perfect linearity of magnetic resonance signals from 1H and the concentration of the contrast agent, making it difficult to obtain images that enable quantitative analysis in molecular imaging and the like. As for nuclides other than proton, 19F nuclei, which are almost equal in sensitivity to proton, are being studied with a view toward molecular imaging applications using MRI; however, 19F has not yet been in practical use because of problems such as the difficulty in synthesizing fluorine-containing compounds. Moreover, when contrast agents using iron oxide or gadolinium, or contrast agents using atoms such as fluorine, are used, their toxicity must be considered to some extent.

MRI imaging can also be performed by introducing 13C-containing molecules into the subject's body, and then measuring the magnetic resonance signals from 13C; hence, 13C-containing molecules are known to be usable as contrast agents for MRI. The magnetic resonance signals from 13C have a low background level in the subject compared with signals from 1H, and are therefore considered usable in obtaining images used for quantitative evaluations. The magnetic resonance signal from 13C, however, is easily affected by the structure of the molecule. Therefore, when a plurality of 13C nuclei are introduced into a single molecule to enhance the magnetic resonance signals from 13C, the chemical shift of each 13C nucleus in the molecule may be dispersed to lower the measurement accuracy. Moreover, attaching a 13C-containing molecule to a protein with a relatively high molecular weight such as an antibody may cause attenuation of magnetic resonance signals from 13C.

In addition, MRI imaging has been required to obtain magnetic resonance images in a short period of time to, for example, lessen the burden on the subject; therefore, the use of molecules with a suitably short T1 relaxation time (longitudinal relaxation) as MRI contrast agents is considered effective. However, when acquiring magnetic resonance signals using a 13C-containing molecule, the T1 relaxation time largely depends on the molecular structure and the like; nevertheless, molecules of a structure that has a short T1 relaxation time and is effective in continuously obtaining magnetic resonance images in a short period of time have been unknown.

In view of the above-described prior art, the development of a technique that is highly safe, usable for quantitative evaluations, and capable of continuously acquiring magnetic resonance signals in a short period of time has been desired.

WO 98/57578 A1 relates to a method employing a tissue-directed ¹³C,¹³C-¹³C,¹³C-¹⁵N or ¹³C-¹³C-¹⁵N isotopically enriched protein reagent as a diagnostic contrasting agent in magnetic resonance imaging to enhance the contrast of a targeted site in a mammalian tissue.

WO 96/07670 A1 relates to poly(ethyene glycol) mixed carbonates which are synthesized by conversion of polyethylene glycol first to the chloroformate then by reaction with the hydroxyl group of N-hydroxybenzotriazole or 2-hydroxypyrimidine or N-hydroxy-2-pyrrolidone. The structural formulae for these mixed carbonates is said to allow for them to smoothly react with the amino groups in aminoglycans and protein and amino containing surface to form stable, hydrolysis resistant carbamate linkages.

J. Am. Chem. Soc. 1998, 120, 10453-10462 concerns the evolution of the density matrix under the average Hamiltonian established under the 2D-MELODRAMA pulse sequence, and closed-form expressions for the dependence of cross-peak intensity as a function of the dipolar mixing time.

### DISCLOSURE OF THE INVENTION

### PROBLEM TO BE SOLVED BY THE INVENTION

It is an object of the invention to provide a magnetic resonance imaging method using a contrast agent that is safe and quantitative, and capable of continuously acquiring magnetic resonance signals with a short repetition time, and to provide a method for acquiring magnetic resonance signals.

### MEANS FOR SOLVING THE PROBLEM

The present inventors conducted extensive research to solve the aforementioned object, and found that the use of a contrast agent comprising a polyethylene glycol containing 13C in a proportion higher than the natural abundance, or a compound labeled with the polyethylene glycol, allows magnetic resonance signals from 13C to be quantitatively measured continuously by repeated application of excitation pulses, with a repetition time of 60 seconds or less (preferably 1 second or less, and more preferably 100 milliseconds or less), and thereby obtain magnetic resonance images usable for quantitative analysis in a short period of time. The present invention was accomplished based on this finding and further improvements thereto.

One aspect of the invention provides a magnetic resonance imaging method as defined below.

Item 1. A magnetic resonance imaging method comprising applying, to a subject administered with a magnetic resonance contrast agent comprising a polyethylene glycol containing 13C in a proportion higher than the natural abundance, or a compound labeled with the polyethylene glycol, pulses of an excitation magnetic field with a repetition time of 60 seconds or less, thereby continuously acquiring magnetic resonance signals to obtain a image.

Item 2. The magnetic resonance imaging method according to Item 1, wherein the proportion of 13C in the polyethylene glycol is from 20 to 100% of the total carbon atoms.

Item 3. The magnetic resonance imaging method according to Item 1 or Item 2, wherein the polyethylene glycol has a weight average molecular weight of 470 to 10,000,000.

Item 4. The magnetic resonance imaging method according to any one of Items 1 to 3, wherein the compound is an antibody labeled with the polyethylene glycol containing 13C in a proportion higher than the natural abundance.

Still another aspect of the invention provides a method for acquiring magnetic resonance signals as defined below.

Item 5. A method for acquiring magnetic resonance signals, comprising applying, to a subject administered with a magnetic resonance contrast agent comprising a polyethylene glycol containing 13C in a proportion higher than the natural abundance, or a compound labeled with the polyethylene glycol, pulses of an excitation magnetic field with a repetition time of 60 seconds or less, thereby continuously acquiring magnetic resonance signals.

Item 6. The method according to Item 5, wherein the proportion of 13C in the polyethylene glycol is from 20 to 100% of the total carbon atoms.

Item 7. The method according to Item 5 or Item 6, wherein the polyethylene glycol has a weight average molecular weight of 470 to 10,000,000.

Item 8. The method according to any one of Items 5 to 7, wherein the compound is an antibody labeled with the polyethylene glycol containing 13C in a proportion higher than the natural abundance.

Yet another aspect of the invention provides the use of a polyethylene glycol containing 13C in a proportion higher than the natural abundance, or a compound labeled with the polyethylene glycol, as defined below.

Item 9. Use of a polyethylene glycol containing 13C in a proportion higher than the natural abundance, or a compound labeled with the polyethylene glycol, in a method for continuously acquiring magnetic resonance signals to obtain a image wherein pulses of an excitation magnetic field are applied with a repetition time of 60 seconds or less.

Item 10. The use according to Item 9, wherein the proportion of 13C in the polyethylene glycol is from 20 to 100% of the total carbon atoms.

Item 11. The use according to Item 9 or Item 10, wherein the polyethylene glycol has a weight average molecular weight of 470 to 10,000,000.

Item 12. The use according to any one of Items 9 to 11, wherein the compound is an antibody labeled with the polyethylene glycol containing 13C in a proportion higher than the natural abundance.

### EFFECTS OF THE INVENTION

The contrast agent employed in the method of the present invention makes it possible to acquire highly accurate magnetic resonance signals even when excitation pulses are applied with a repetition time of 60 seconds or less (preferably 1 second or less, more preferably 250 milliseconds or less, and particularly preferably 100 milliseconds or less), and is therefore useful in obtaining sharp magnetic resonance images at high speed.

Even though the polyethylene glycol for use in the contrast agent employed in the method of the invention contains a plurality of 13C nuclei, the chemical shift of each 13C nucleus is not dispersed and concentrates on one chemical shift, allowing the acquisition of highly accurate magnetic resonance signals. In addition, the contrast agent employed in the method of the invention utilizes magnetic resonance signals from 13C, which have a low background level in the subject compared with signals from 1H, thus allowing the acquisition of images that enable quantitative evaluations.

Moreover, the polyethylene glycol containing 13C in a proportion higher than the natural abundance, even when it is attached to other high-molecular-weight compounds such as proteins and the like, hardly affects the magnetic resonance signals. Accordingly, the present invention enables, for example, the diagnosis, determination, and visualization as described in the following Items (1) to (4) to be performed in a short period of time.
(1) The polyethylene glycol is attached to an antibody that specifically recognizes a specific lesion, and the resulting compound is used as a contrast agent to visualize the lesion to make a diagnosis.
(2) The polyethylene glycol is attached to an antibody that specifically recognizes specific cells, and the resulting compound is used as a contrast agent to visualize the dynamics of the cells in vivo.
(3) The polyethylene glycol or a compound having the polyethylene glycol attached thereto is incorporated into a DDS preparation such as a liposome preparation, and the resulting preparation is administered to determine the degree of accumulation of the preparation in the target site.
(4) A polyethylene glycol containing 13C is directly administered to a human to allow the polyethylene glycol to accumulate in a specific organ or site for a certain period of time, and thereby visualize the specific organ or site.

Furthermore, because the contrast agent employed in the method of the invention uses 13C, it is highly safe and stable even after time has passed, compared with contrast agents containing radioactive compounds as used in PET, SPECT, etc.; therefore, the contrast agent advantageously allow a lengthy amount of time for magnetic resonance imaging.

### BEST MODE FOR CARRYING OUT THE INVENTION

The contrast agent employed in the method of the invention comprises a polyethylene glycol containing 13C in a proportion higher than the natural abundance (hereinafter "13C-PEG"), or a compound labeled with the 13C-PEG.

13C-PEG for use in the invention may be any that contains 13C in a proportion higher than the natural abundance (i.e., about 1% or more of the total carbon atoms). In order to enhance the detection sensitivity of magnetic resonance signals, the proportion of 13C in the total carbon atoms is from 20 to 100%, preferably 50 to 100%, more preferably 90 to 100%, and particularly preferably nearly 100%. Polyethylene glycol is composed of the repeating unit -CH₂CH₂O-, and has the same chemical environment for all of the carbon atoms. Therefore, polyethylene glycol is advantageous in that, even if there are a plurality of 13C nuclei in one molecule, the chemical shift of each 13C nucleus is not dispersed and concentrates on one chemical shift, allowing the detection of enhanced magnetic resonance signals.

The molecular weight of 13C-PEG for use in the invention is not limited, and may be set suitably according to the proportion of 13C and the like. For example, when the proportion of 13C is low, the molecular weight of 13C-PEG is preferably high, whereas when the proportion of 13C is high, the molecular weight of 13C-PEG may be low. One example of 13C-PEG for use in the invention is 13C-PEG with a weight average molecular weight of 470 to 10,000,000, and preferably 6,000 to 2,000,000.

While the above-described 13C-PEG may be used by itself, a compound labeled with the 13C-PEG (hereinafter a "13C-PEG modified compound") may also be used. The term "13C-PEG modified compound" here denotes a compound to which 13C-PEG is attached directly or via a linker group. In such 13C-PEG modified compounds, examples of compounds labeled with (attached) 13C-PEG include antibodies such as monoclonal antibodies and polyclonal antibodies; the Fab fragments of these antibodies; serum proteins such as albumin and transferrin; pharmacologically active proteins such as interferon, erythropoietin, interleukin, M-CSF, G-CSF, insulin, and adipokine; low-molecular compounds such as EP-1873 (Epix Pharma), Evans Blue, Congo red, thioflavin-S, (E,E)-1-bromo-2,5-bis(3-hydroxycarbonyl-4-hydroxy)styrylbenzene (BSB), and (E,E)-1-fluoro-2,5-bis(3-hydroxycarbonyl-4-hydroxy)styrylbenzene (FSB); compounds that form liposomes capable of enclosing pharmaceuticals; etc. For example, a 13C-PEG modified compound having an antibody capable of specifically binding to a specific lesion (such as, for example, cancer, arteriosclerosis, or inflammation) attached thereto can visualize the specific lesion. In addition, the use of a 13C-PEG modified compound having a pharmacologically active protein attached thereto enables the degree of accumulation of the pharmacologically active protein in the target site to be treated.

The 13C-PEG modified compound is prepared by attaching 13C-PEG to a compound to be labeled, according to a known process. When the compound to be labeled has an amino group (more specifically, when the compound is an antibody or a pharmacologically active protein), one suitable example of a process includes converting the polyethylene glycol to an activated ester using N-hydroxysuccinimide (NHS) to form an amide bond with the compound to be labeled.

In the 13C-PEG modified compound, the number of 13C-PEGs attached to the compound to be labeled is not limited as long as the desired activity of the compound to be labeled is not impaired. For example, the 13C-PEG modified compound may have one or more 13C-PEGs attached to the compound to be labeled.

The contrast agent employed in the method of the invention is prepared by dissolving the 13C-PEG or 13C-PEG modified compound in a pharmacologically or chemically acceptable solvent such as a saline solution, an isotonic phosphate buffer, or the like. The concentration of the polyethylene glycol or 13C-PEG modified compound in the contrast agent can be suitably adjusted according to the image formation method, measurement method, site to be measured, and the like. For example, the concentration of the 13C-PEG or 13C-PEG modified compound may be from 0.0001 to 100% by weight, preferably 0.001 to 50% by weight, and more preferably 0.01 to 10% by weight, based on the total amount of the contrast agent.

The contrast agent employed in the method of the invention may further comprise, in addition to the above-described components, additives such as a solubilizer, an emulsifier, a viscosity modifier, a buffer, and the like.

The contrast agent is administered to a subject intravenously, subcutaneously, intramuscularly, orally, or via other routes. The dose of the contrast agent is adjusted suitably according to the 13C content of the 13C-PEG or 13C-PEG modified compound, the site to be measured using magnetic resonance imaging, and the like. For example, the dose of the contrast agent may be adjusted so that the number of 13C atoms of the 13C-PEG or 13C-PEG modified compound at the site to be measured is from 1x10⁻¹² mol or more, preferably 1x10⁻⁸ mol or more, and more preferably 1x10⁻⁶ mol or more, per 1 cm³.

The contrast agent employed in the method of the invention is used to continuously acquire magnetic resonance signals by applying pulses of an excitation magnetic field (RF waves) with a repetition time of 60 seconds or less. The term "repetition time" (TR) here refers to the total length of time required for a single pulse sequence. Specifically, TR refers to the time interval from the beginning of a pulse sequence to the beginning of the next pulse sequence in the repetitive acquisition of the resonance signal. The 13C-PEG or 13C-PEG modified compound used in the contrast agent employed in the method of the invention advantageously exhibits a suitably short T1 relaxation time, thus allowing magnetic resonance images to be continuously acquired, with the repetition time set as short as described above. In order to continuously acquire magnetic resonance signals at an even higher speed, the contrast agent employed in the method of the invention enables the repetition time to be set to preferably 1 second or less, more preferably 250 milliseconds or less, and particularly preferably from 60 to 100 milliseconds. The contrast agent thus enables a short repetition time and the continuous acquisition of magnetic resonance signals, making it suitable for use in high-speed imaging.

The magnetic resonance signals acquired using the contrast agent can be used directly for a diagnosis and the like. The magnetic resonance signals can also be converted to magnetic resonance images, which can be used for various diagnoses.

Other conditions for acquiring magnetic resonance signals using the contrast agent employed in the method of the invention, such as the pulse duration time of an excitation magnetic field or the method of magnetic resonance signal measurement, can be suitably selected from the conditions generally employed to acquire magnetic resonance signals. For magnetic resonance signal imaging, conditions can be suitably selected from those generally employed to obtain magnetic resonance images.

Accordingly, the contrast agent of the invention can be applied to known imaging methods and, more specifically, methods such as chemical shift imaging, proton detection 13C chemical shift imaging, fast spin echo method, gradient echo method, and the like.

### EXAMPLES

The present invention will be described in detail below with reference to the Examples; however, the invention is not limited by these Examples. In the following Examples, the proportion (%) given before the notation "13C-PEG" refers to the proportion of 13C in the 13C-PEG per total carbon atoms. The numerical value given after the notation "13C-PEG" refers to the molecular weight of the 13C-PEG.

### Example 1

The following experiments were conducted to examine the NMR spectral characteristics of 13C-PEGs. 13C-PEG6000 (hereinafter "99%13C-PEG6000", purchased from Cambridge Isotope Laboratories, Inc. (CIL)), in which nearly all of the carbon atoms are 13C, was dissolved in heavy water (D₂O) to a concentration of 2.2 mg/ml, and the NMR spectrum of the resulting sample was measured. In addition, 13C-PEG6000 containing 13C at natural abundance (1%) (hereinafter "1%13C-PEG6000") was dissolved in heavy water (D₂O) to a concentration of 2.2 mg/ml, and the NMR spectrum of the resulting sample was measured.

The NMR spectrometer and measurement conditions were as follows.
System: a high-resolution NMR spectrometer
Console: Varian Unity INOVA
Magnet: Oxford 300 MHz
Measurement conditions: observed frequency: 75 MHz, measured temperature: 23°C, a single-pulse method (proton decoupling), acquisition delay: 1 sec., measured with 45° pulses

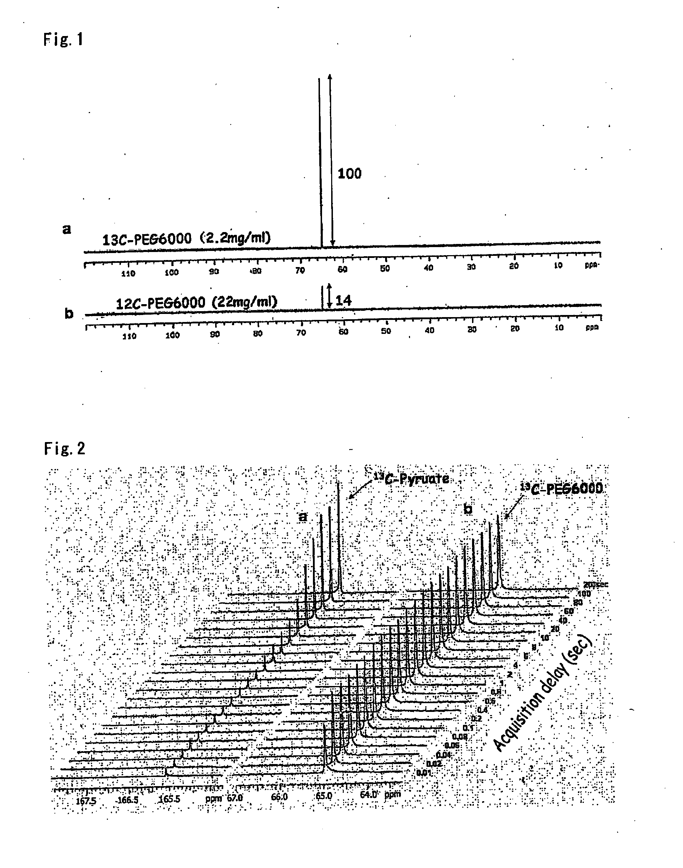

The results are shown in FIG. 1. Although 99%13C-PEG6000 is a macromolecule, it exhibited a very sharp NMR signal (see FIG. 1a). In addition, 99%13C-PEG6000 has the same chemical environment for all of the carbon atoms, allowing their chemical shifts to concentrate on one point, resulting in a high signal intensity. On the other hand, 1%13C-PEG6000 with a concentration 10-fold higher than that of 99%13C-PEG6000 exhibited a signal intensity about one-tenth that of 99%13C-PEG6000. This confirmed that the signal intensity derived from 13C is commensurate with the number of 13C nuclei, and is extremely quantitative.

### Example 2

99%13C-PEG6000 was dissolved in heavy water (D₂O) solvent to a concentration of 2.5 mg/ml, and using the resulting sample, the effect of reducing a interval of the acquisition delay that follows pulse radiation (90° pulses) and FID acquisition (1.3 sec.) (the time required from the completion of the echo acquisition time to the next excitation; dead time; acquisition delay) on the signal intensity was examined under the measurement conditions shown below. For comparison, 13C-pyruvic acid (sodium pyruvate (1-¹³C, 99%), from CIL) was dissolved in heavy water to a concentration of 25 mg/ml, and a glucose in which the 1-position carbon is 13C (D-Glucose (1-¹³C, 99%), from CIL; hereinafter "13C-glucose") was dissolved in heavy water to a concentration of 2.2 mg/ml. Each of these resulting solutions was tested as samples in the same manner as above.
System: a high-resolution NMR spectrometer
Console: Varian Unity INOVA
Magnet: Oxford 300 MHz
Measurement conditions: observed frequency: 75 MHz, measured temperature: 23°C, a single-pulse method (proton decoupling), measured with 45° pulses

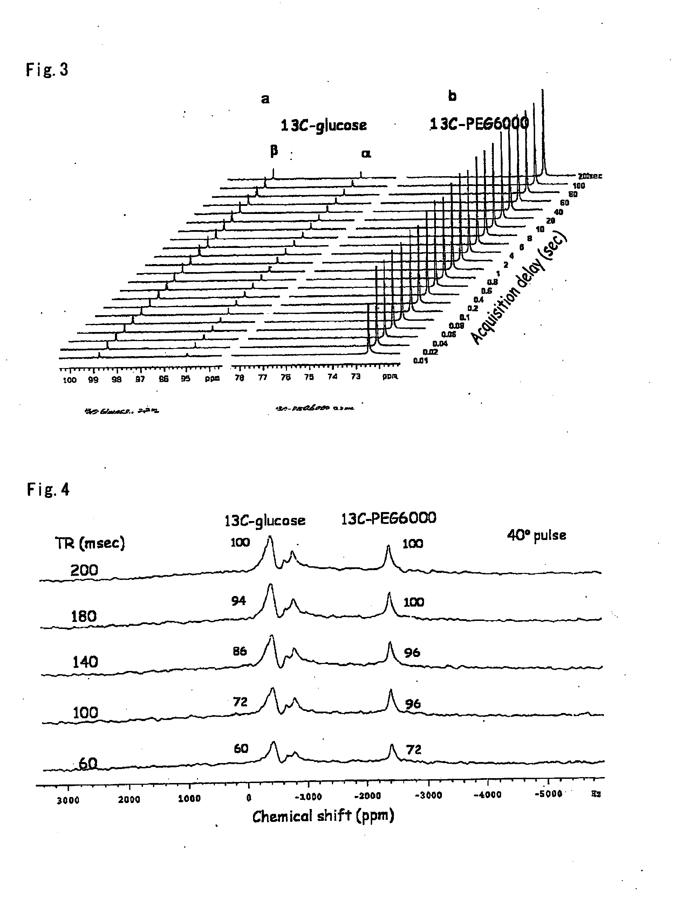

The results are shown in FIGS. 2 and 3. As shown in FIG. 2a, with 13C-pyruvic acid, the signal intensity decreases abruptly by reducing the acquisition delay to 60 seconds or less. This is because the T1 relaxation time of 13C-pyruvic acid is very long (the T1 relaxation time of carbon to which protons are not directly attached is long). ,In contrast, with 99%13C-PEG6000, as shown in FIGS. 2b and 3b, even though the acquisition delay interval was reduced to about 20 msec., the signal intensity hardly decreased. This is believed to be because the T1 relaxation time of 99%13C-PEG6000 is relatively short.

With 13C-glucose, signals for the carbon of both the α-and β-isomers of the glucose were observed. Because the carbon of both the isomers has protons directly covalently bonded thereto, the T1 times of these isomers are shorter than that of the pyruvic acid. Hence, although the acquisition delay interval was reduced, there was not an abrupt decrease as observed in the pyruvic acid at an interval of 60 seconds or less. Nevertheless, the signal intensities for the 1-position carbon of both the α-and β-isomers showed decreases due to the shortened acquisition delay intervals (see FIG. 3a). The sum of the signal intensities of the carbon of both the α- and β-isomers of the glucose showed a 21% decrease when the acquisition delay interval was reduced from 200 seconds to 20 milliseconds, whereas the signal intensity of 99%13C-PEG6000 only showed a decrease as small as 3.9%. Therefore, the phenomenon observed in 99%13C-PEG6000, that the signal intensity does not decrease by reducing the acquisition delay interval to 20 milliseconds, is believed to be due to the T1 relaxation time characteristic of the 13C-PEG.

### Example 3

99%13C-PEG6000 was dissolved in a heavy water (D₂O) solvent to a concentration of 2.5 mg/ml, and using the resulting sample, the effect of pulse application with a repetition time of 60 to 200 milliseconds on signal intensity was examined using an MRI system at a field strength of 7 Tesla, under the conditions shown below. For comparison, 13C-glucose was dissolved in heavy water to a concentration of 2.2 mg/ml, and the resulting sample was similarly tested.
System: an MRI system (field strength: 7 Tesla)
Console: Varian Unity INOVA
Magnet: JASTEC 7T
Measurement conditions: observed frequency: 75 MHz, measured temperature: 23°C, a single-pulse method (proton decoupling), measured with 40° pulses

The results are shown in FIG. 4. As is clear from FIG. 4, in the measurements using 40° pulses, the signal intensity of the glucose showed a decrease of about 30% when the pulse interval was reduced from 200 milliseconds to 100 milliseconds, whereas the signal intensity of 99%13C-PEG6000 showed a decrease of only about 4% even when the pulse interval was reduced to 100 milliseconds. These results revealed that also in an MRI system, the ability of 13C-PEG6000 to shorten the repetition time can be utilized.

### Example 4

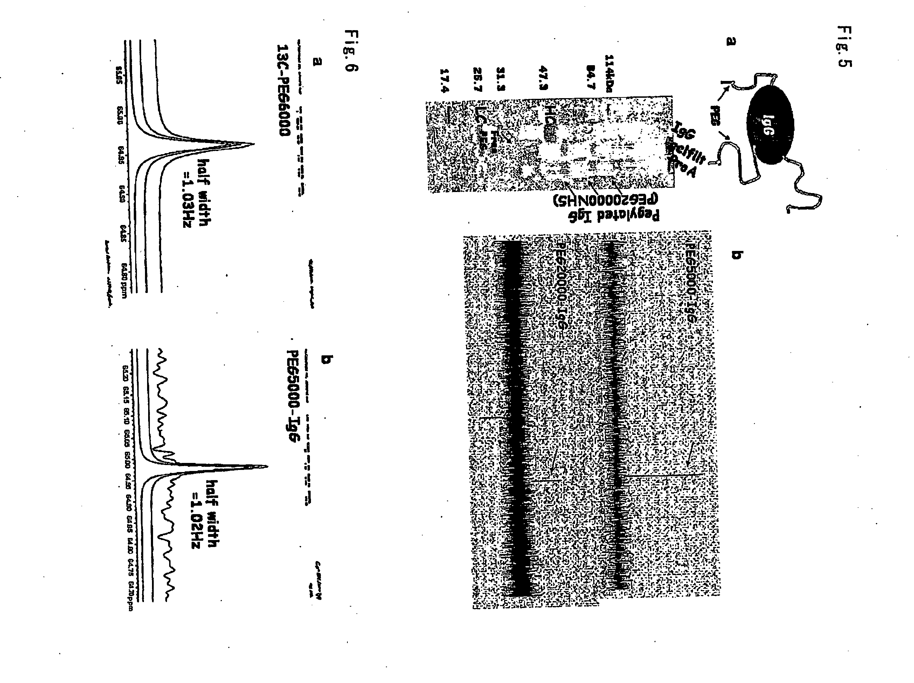

IgG was labeled with each one of 1%13C-PEG5000NHS and 1%13C-PEG20000NHS (both from Nippon Oil & Fats Co., Ltd.), which were obtained by converting one terminal hydroxyl group of 1% 13C-PEG to a NHS group. After the labeling reaction, the resulting product was subjected to purification steps using gel filtration and a Protein A column to thereby remove unreacted 1%13C-PEG (see FIG. 5a). As is clear from the image of SDS-PAGE shown in FIG. 5a, several bands were observed on the high molecular weight range, confirming that 1%13C-PEG had been actually labeled to IgG via a covalent bond.

The thus-obtained 1%13C-PEG5000-labeled IgG was dissolved in heavy water to a concentration of 14.1 mg/ml, and the 1%13C-PEG20000-labeled IgG was dissolved in heavy water to a concentration of 5.1 mg/ml. The NMR spectrum of each of the resulting samples was then measured. The NMR spectrometer and measurement conditions were as follows.
System: a high-resolution NMR spectrometer
Console: Varian Unity INOVA
Magnet: Oxford 300 MHz
Measurement conditions: observed frequency: 75 MHz, measured temperature: 23°C, a single-pulse method (proton decoupling), acquisition delay: 1 sec., measured with 45° pulses

The results are shown in FIGS. 5b and 6. As is clear from FIG. 5b, it was confirmed that 1%13C-PEG5000 and 1%13C-PEG20000 attached to IgG, as with the cases not attached to IgG, exhibited very sharp signals that concentrated on one chemical shift. In addition, as can be seen from FIG. 6, the half-width of the signal of 1%13C-PEG5000 was hardly affected by attaching 1%13C-PEG5000 to IgG. These results revealed that attaching PEGs to macromolecular proteins such as IgG does not cause problems such as reduced PEG signal intensity, broadened spectra, etc.

### Example 5

The change in the intensity and half-width of an NMR signal along with an increase in the PEG molecular weight was examined using PEGs containing 13C at natural abundance (1%). Three types of PEGs, with average molecular weights of 35,000, 500,000, and 2,000,000, were used. NMR spectral measurements were conducted using a high-resolution nuclear magnetic resonance spectrometer. The measurement conditions were as follows.
System: JEOL JNM-ECA500
Magnet: Oxford (11.7 Tesla, 500 MHz)

### Measurement conditions

Observed frequency: 125 MHz
Temperature: 25°C
Observed width: 31 KHz
Data point: 32 K
Pulse sequence: single-pulse decoupling
Flip angle: 45°
Acquisition delay: 2 sec.
Data acquisition time: 1 sec.

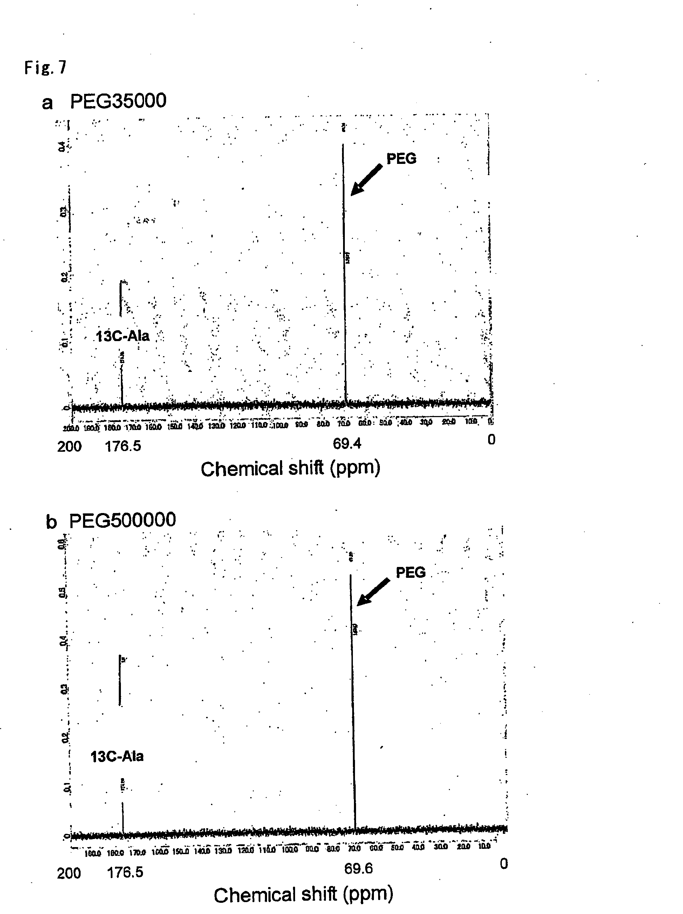

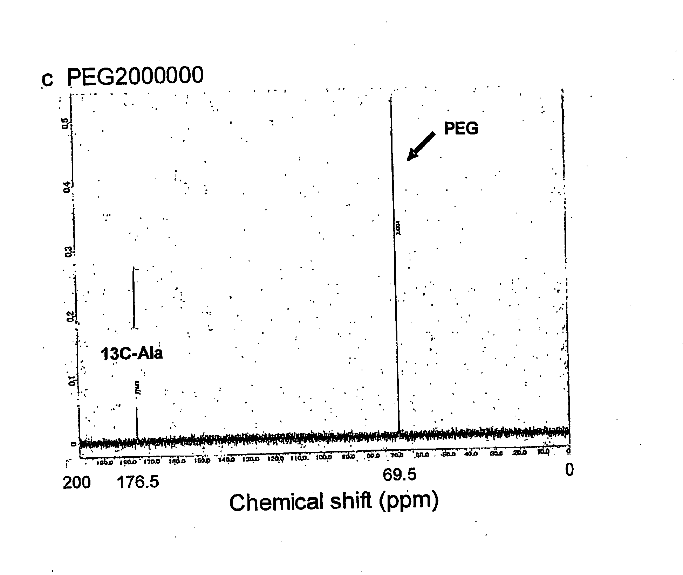

The results are shown in FIG. 7. All of the PEGs with different molecular weights were 0.5 mg/ml in concentration (solvent: D₂O). 0.5 mM 13C-alanine (from CIL, the carbon of the carboxylic acid is 13C) was added to each sample as an internal control, and then measurements were conducted. The signal from the carbon of the carboxylic acid of 13C-alanine was observed at 176.5 ppm, and the signals from all of the PEGs with different molecular weights were observed near 69.5 ppm (with a deviation of about 0.1 ppm depending on the molecular weight). The signal of each PEG was very sharp, and measurement of the half-width of the NMR signal of each PEG revealed that PEG35000, PEG500000, and PEG2000000 exhibited half-widths of 2.99, 3.03, and 3.25 Hz, respectively, showing that the half-width hardly changed even though the molecular weight increased. When the intensity of the NMR signal of each PEG was evaluated in terms of its signal height, assuming the signal of the carboxylic acid of 13C-alanine to be 1, each of the PEGs exhibited a signal intensity (a peak height) of from about 8 to about 10, and there was no significant difference in signal intensity (peak height) due to the molecular weight differences between the sample PEG solutions with the same weight concentration (FIGS. 7a, 7b, and 7c). Specifically, it was experimentally demonstrated that even though the PEG has a molecular weight as high as about 2,000,000, the intensity of the NMR signal from the carbon in the molecule is not at all attenuated. In terms of molar concentration, the concentrations of the PEG35000, PEG500000, and PEG2000000 samples were 14.2 µM, 1.0 µM, and 0.25 µM, respectively. When the signal height per molecule of each PEG was evaluated based on these molar concentrations, assuming the signal height of 13C-alanine to be 1, the signal heights of PEG35000, PEG500000, and PEG2000000 were calculated to be 283, 4,000, and 19,400, respectively. The result shows that the signal intensity (peak height) increased substantially proportionately with the molecular weight of the PEG. These results revealed that the signal intensity (peak height) of the NMR signal of PEG increases substantially proportionately with the molecular weight of the PEG, as long as the viscosity of the solution need not be considered.

### Example 6

99%13C-PEG6000 was dissolved in pure water (H₂O) to a concentration of 33 mg/ml, and the solution was used as a sample. 0.1 ml of the sample was injected into the temporalis muscle of rats (14-week-old male SD rats, purchased from CLEA Japan), and MRI images were obtained under the following conditions.
System: MR console Varian Unity INOVA, magnet: JASTEC 7T
Pulse sequence: proton decoupled 13C 2D chemical shift imaging

### (no slice selection)

Encoding phase: 8x8
Photographing field (FOV): 50x50 mm²
Repetition time: 1 sec.
Matrix: 32×32
Number of accumulation: eight
Total measurement time: 8 min., 32 sec.

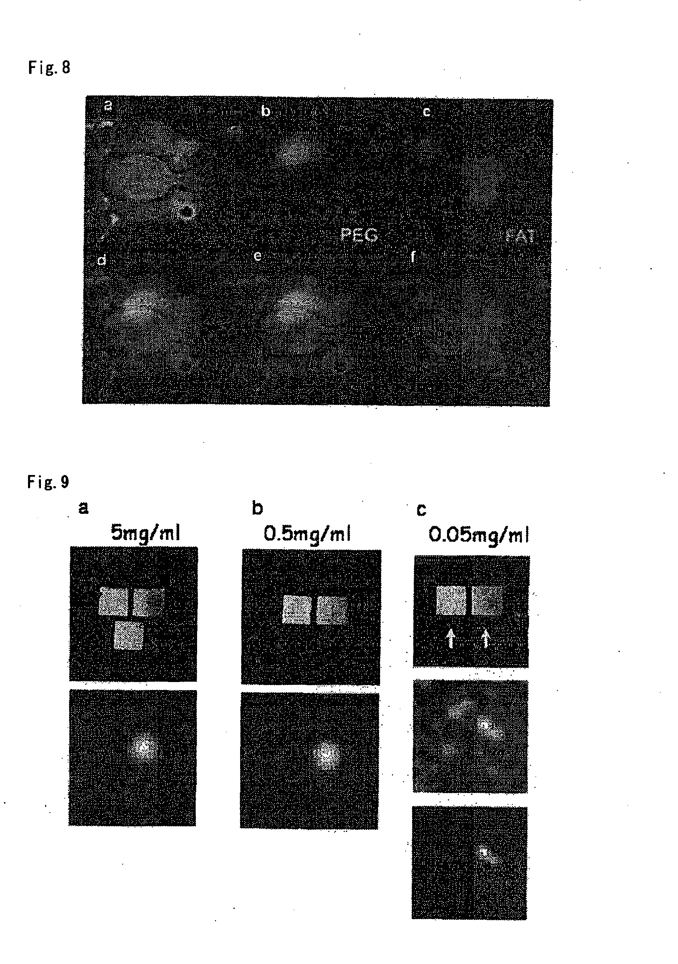

The results are shown in FIG. 8. These results confirmed that 99%13C-PEG6000 makes it possible to obtain clear images in the temporalis muscle of rats and visualize them, even when the repetition time is as short as 1 second.

### Example 7

99%13C-PEG6000 was dissolved in a saline solution to 0.05 mg/ml, 0.5 mg/ml, or 5 mg/ml, and 1 ml each of these samples was added to 1 cm square cuvettes. MRI images of the cuvettes containing each solution of 99%13C-PEG6000 were obtained under the conditions shown below. For comparison, MRI images were similarly obtained using a saline solution containing 10 wt% 13C-glucose or a saline solution alone.
System: MR console Varian Unity INOVA, magnet: JASTEC 7T
Pulse sequence: proton decoupled 13C 2D chemical shift imaging

### (no slice selection)

Encoding phase: 8x8
Photographing field (FOV): 50×50 mm²
Matrix: 32×32
Repetition time: 250 millisec.
Number of accumulation: 128
Total measurement time: 34 min.

The results are shown in FIG. 9. As can be seen from FIG. 9, the 99%13C-PEG6000 solutions at 5 mg/ml and 0.5 mg/ml contained in 1 cm square cuvettes were visualized with a sufficient contrast. On the other hand, the 99%13C-PEG6000 solution at 0.05 mg/ml showed a considerable decrease in SN ratio (signal-to-noise ratio), but was nevertheless visualized to a degree such that the positions of the cuvettes containing 99%13C-PEG6000 could sufficiently be observed.

### Example 8

Using aqueous solutions of 99%13C-PEG6000, MRI images were obtained using some imaging methods, and the resulting images were compared. More specifically, 99%13C-PEG6000 was dissolved in pure water (H₂O) to a concentration of 30 mg/ml or 5 mg/ml, and 1 cm³ cuvettes were charged with one of these solutions, and then images thereof were obtained. Four types of imaging methods, i.e., 13C chemical shift imaging (13C-CSI), proton detection 13C chemical shift imaging (1H-detected 13C-CSI), 13C gradient echo (13C-GRE), and 13C fast spin echo (13C-FSE), were employed. Imaging using each of these methods was performed under the following conditions.
13C-CSI: matrix: 8x8, FOV: 50×50 mm², repetition time: 1 sec., measurement time: 128 sec.
1H-detected 13C-CSI: matrix: 8x8, FOV: 50×50 mm², repetition time: 1 sec., measurement time: 128 sec.
13C-GRE: matrix: 64x64, FOV: 50×50 mm², repetition time: 30 msec., measurement time: 123 sec., proton decoupling
13C-FSE: matrix: 32x32, FOV: 50×50 mm², repetition time: 1 sec., echo train: 8, echo space: 5 msec., centric acquisition, measurement time: 64 sec., proton decoupling

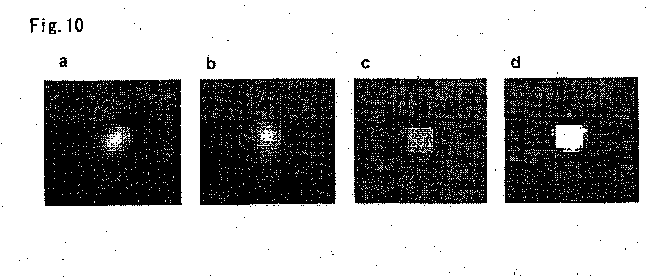

The results are shown in FIG. 10. FIG. 10a shows an image obtained using the 13C chemical shift imaging method (13C-CSI); FIG. 10b shows an image obtained using the proton detection 13C chemical shift imaging method (1H-detected 13C-CSI); FIG. 10c shows an image obtained using the 13C gradient echo method (13C-GRE); and FIG. 10d shows an image obtained using the 13C fast spin echo method (13C-FSE). Each of the images shown in FIGS. 10a to 10d includes an upper cuvette charged with the 5 mg/ml solution of 99%13C-PEG6000 and a lower cuvette charged with the 30 mg/ml solution of 99%13C-PEG6000.

While each of the cuvettes charged with the 5 mg/ml or 30 mg/ml solution of 99%13C-PEG6000 was visualized, the 30 mg/ml solution was confirmed to be visualized more clearly under the short-period imaging conditions as in this case. A comparison between 13C-CSI (FIG. 10a) and 1H-detected 13C-CSI (FIG. 10b) did not show a significant difference in terms of SN ratio, resolution, and the like. On the other hand, the images obtained using 13C-GRE and 13C-FSE allowed one to clearly recognize the square shape of the cuvettes. These results confirmed that measurements using 13C-GRE and 13C-FSE allow the acquisition of high-resolution images in about the same or even a shorter integral time, compared with 13C-CSI and 1H-detected 13C-CSI. The foregoing results demonstrate that when 13C-PEG is used as a contrast agent, high resolution images can be acquired in a shorter time by suitably applying a method such as 13C-GRE or 13C-FSE.

### BRIEF DESCRIPTION OF THE DRAWINGS

FIG. 1 is a diagram showing the 13C-NMR spectra of 99%13C-PEG6000 (2.2 mg/ml) and 1%13C-PEG6000 (22 mg/(ml)) measured in Example 1.
FIG. 2 is a diagram showing the results of the measurements made in Example 2, i.e., the relationship between the 13C-NMR signal intensity and the acquisition delay for each of 13C-pyruvic acid and 99%13C-PEG6000.
FIG. 3 is a diagram showing the results of the measurements made in Example 2, i.e., the relationship between the 13C-NMR signal intensity and the acquisition delay for each of 13C-glucose and 99%13C-PEG6000.
FIG. 4 is a diagram showing the results of the measurements made in Example 3, i.e., the 13C-NMR signal intensities of 13C-glucose and 99%13C-PEG6000 when the repetition time was varied from 60 to 960 milliseconds, using 40° pulses.
FIG. 5a shows a photograph of SDS polyacrylamide gel electrophoresis (SDS-PAGE) of 1%13C-PEG20000-labeled IgG samples obtained in various purification steps, wherein the leftmost lane shows the molecular weight marker, the subsequent lane shows unlabeled IgG ("IgG" in the figure), the subsequent lane shows 1%13C-PEG20000-labeled IgG gel-filtrated (Sephacryl S-200; Pharmacia) after the labeling reaction ("gelfilt" in the figure), and the subsequent lane shows 1%13C-PEG20000-labeled IgG obtained by affinity-purification of the gel-filtrated sample using a Protein A column ("Pro A" in the figure); and FIG. 5b shows the results of the 13C-NMR spectra of 1%13C-PEG5000-labeled IgG and 1%13C-PEG20000-labeled IgG measured in Example 4.
FIG. 6 shows diagrams comparing the half-widths of the signals of 99%13C-PEG6000 and 1%13C-PEG5000-labeled IgG.
FIG. 7 shows diagrams showing the NMR spectra obtained in Example 5, wherein FIG. 7a shows the NMR spectrum of 1%13C-PEG35000, 0.5 mg/ml (14.2 µM); FIG. 7b shows the NMR spectrum of 1%13C-PEG500000, 0.5 mg/ml (1.0 µM); and FIG. 7c shows the NMR spectrum of 1%13C-PEG2000000, 0.5 mg/ml (0.25 µM).
FIG. 8 shows MRI images obtained in Example 6, wherein FIG. 8a shows a proton image; FIG. 8b shows a 13C-chemical shift image of 99%13C-PEG6000, displayed in blue; FIG. 8c shows a 13C-chemical shift image of the endogenous fat of the temporalis muscle of the rat, displayed in red; FIG. 8d displays the 13C-chemical shift image of 99%13C-PEG6000 and the 13C-chemical shift image of the endogenous fat, superimposed over the proton image; FIG. 8e displays the 13C-chemical shift image of 99%13C-PEG6000 superimposed over the proton image; and FIG. 8f shows the 13C-chemical shift image of the internal fat superimposed over the proton image.
FIG. 9 shows MRI images obtained in Example 7, wherein FIG. 9a shows an image of 5 mg/ml of 99%13C-PEG6000. (The upper photograph is a proton image; the upper left cuvette contains 10 wt% 13C-glucose, the upper right cuvette contains 5 mg/ml of 13C-PEG6000, and the lower cuvette contains a saline solution. The lower photograph is a CSI image of 13C-PEG6000.) FIG. 9b shows an image of 0.5 mg/ml of 99%13C-PEG6000. (The upper photograph is a proton image; the left cuvette contains a saline solution, and the right cuvette contains 0.5 mg/ml 13C-PEG6000. The lower photograph is a CSI image of 13C-PEG6000.) FIG. 9c shows an image of 0.05 mg/ml of 99%13C-PEG6000. (The upper photograph is a proton image; the left cuvette contains a saline solution, and the right cuvette contains 0.05 mg/ml of 99%13C-PEG6000. The middle photograph is a CSI image of 99%13C-PEG6000. The lower photograph is an image obtained by cutting down the noise level of the middle image using image processing; the image allows one to clearly recognize the presence of 0.05 mg/ml of 99%13C-PEG6000.)
FIG. 10 shows MRI images obtained in Example 8, wherein FIG. 10a is an image obtained using the 13C chemical shift imaging method (13C-CSI); FIG. 10b is an image obtained using the proton detection 13C chemical shift imaging method (1H-detected 13C-CSI); FIG. 10c shows an image obtained using the 13C gradient echo method (13C-GRE); and FIG. 10d shows an image obtained using the 13C fast spin echo method (13C-FSE); in each of the images of 10a to 10d, two cuvettes are placed vertically, the upper cuvette containing 5 mg/ml of 99%13C-PEG6000, and the lower cuvette containing 30 mg/ml of 99%13C-PEG6000.

## Claims

1. A magnetic resonance imaging method comprising applying, to a subject administered with a magnetic resonance contrast agent comprising a polyethylene glycol containing 13C in a proportion higher than the natural abundance, or a compound labeled with said polyethylene glycol, pulses of an excitation magnetic field with a repetition time of 60 seconds or less, thereby continuously acquiring magnetic resonance signals to obtain an image.

2. The magnetic resonance imaging method according to claim 1, wherein the proportion of 13C in the polyethylene glycol is from 20 to 100% of the total carbon atoms.

3. The magnetic resonance imaging method according to claim 1 or claim 2, wherein the polyethylene glycol has a weight average molecular weight of 470 to 10,000,000.

4. The magnetic resonance imaging method according to any one of claims 1 to 3, wherein the compound is an antibody labeled with the polyethylene glycol containing 13C in a proportion higher than the natural abundance.

5. A method for acquiring magnetic resonance signals, comprising applying, to a subject administered with a magnetic resonance contrast agent comprising a polyethylene glycol containing 13C in a proportion higher than the natural abundance, or a compound labeled with said polyethylene glycol, pulses of an excitation magnetic field with a repetition time of 60 seconds or less, thereby continuously acquiring magnetic resonance signals.

6. The magnetic resonance imaging method according to claim 5, wherein the proportion of 13C in the polyethylene glycol is from 20 to 100% of the total carbon atoms.

7. The magnetic resonance imaging method according to claim 5 or claim 6, wherein the polyethylene glycol has a weight average molecular weight of 470 to 10,000,000.

8. The magnetic resonance imaging method according to any one of claims 5 to 7, wherein the compound is an antibody labeled with the polyethylene glycol containing 13C in a proportion higher than the natural abundance.

9. Use of a polyethylene glycol containing 13C in a proportion higher than the natural abundance, or a compound labeled with the polyethylene glycol, in a method for continuously acquiring magnetic resonance signals to obtain an image, wherein pulses of an excitation magnetic field are applied with a repetition time of 60 seconds or less.

10. The use according to claim 9, wherein the proportion of 13C in the polyethylene glycol is from 20 to 100% of the total carbon atoms.

11. The use according to claim 9 or claim 10, wherein the polyethylene glycol has a weight average molecular weight of 470 to 10,000,000.

12. The use according to any one of claims 9 to 11, wherein the compound is an antibody labeled with the polyethylene glycol containing 13C in a proportion higher than the natural abundance.

## Patentansprüche

1. Magnetresonanz-Bildgebungsverfahren, das umfasst, dass auf eine Testperson, der ein Magnetresonanz-Kontrastmittel verabreicht wurde, das ein Polyethylenglycol, das 13C in einem höheren Anteil als das natürliche Vorkommen enthält, oder eine Verbindung, die mit dem Polyethylenglycol markiert ist, umfasst, Pulse eines Anregungsmagnetfelds mit einer Wiederholungszeit von 60 Sekunden oder weniger ausgeübt werden, wodurch kontinuierlich Magnetresonanz-Signale gewonnen werden, um ein Bild zu erhalten.

2. Magnetresonanz-Bildgebungsverfahren gemäß Anspruch 1, worin der Anteil von 13C im Polyethylenglycol von 20 bis 100 % der gesamten Kohlenstoffatome ist.

3. Magnetresonanz-Bildgebungsverfahren gemäß Anspruch 1 oder 2, worin das Polyethylenglycol ein gewichtsgemitteltes Molekulargewicht von 470 bis 10.000.000 hat.

4. Magnetresonanz-Bildgebungsverfahren gemäß irgendeinem der Ansprüche 1 bis 3, worin die Verbindung ein Antikörper ist, der mit dem Polyethylenglycol, das 13C in einem höheren Anteil als das natürliche Vorkommen enthält, markiert ist.

5. Verfahren zum Gewinnen von Magnetresonanz-Signalen, das umfasst, dass auf eine Testperson, der ein Magnetresonanz-Kontrastmittel verabreicht wurde, das ein Polyethylenglycol, das 13C in einem höheren Anteil als das natürliche Vorkommen enthält, oder eine Verbindung, die mit dem Polyethylenglycol markiert ist, umfasst, Pulse eines Anregungsmagnetfelds mit einer Wiederholungszeit von 60 Sekunden oder weniger ausgeübt werden, wodurch kontinuierlich Magnetresonanz-Signale gewonnen werden.

6. Magnetresonanz-Bildgebungsverfahren gemäß Anspruch 5, worin der Anteil von 13C im Polyethylenglycol von 20 bis 100 % der gesamten Kohlenstoffatome ist.

7. Magnetresonanz-Bildgebungsverfahren gemäß Anspruch 5 oder 6, worin das Polyethylenglycol ein gewichtsgemitteltes Molekulargewicht von 470 bis 10.000.000 hat.

8. Magnetresonanz-Bildgebungsverfahren gemäß irgendeinem der Ansprüche 5 bis 7, worin die Verbindung ein Antikörper ist, der mit dem Polyethylenglycol, das 13C in einem höheren Anteil als das natürliche Vorkommen enthält, markiert ist.

9. Verwendung eines Polyethylenglycols, das 13C in einem höheren Anteil als das natürliche Vorkommen enthält, oder einer Verbindung, die mit dem Polyethylenglycol markiert ist, in einem Verfahren zum kontinuierlichen Gewinnen von Magnetresonanz-Signalen, um ein Bild zu erhalten, worin Pulse eines Anregungsmagnetfelds mit einer Wiederholungszeit von 60 Sekunden oder weniger ausgeübt werden.

10. Verwendung gemäß Anspruch 9, worin der Anteil von 13C im Polyethylenglycol von 20 bis 100 % der gesamten Kohlenstoffatome ist.

11. Verwendung gemäß Anspruch 9 oder 10, worin das Polyethylenglycol ein gewichtsgemitteltes Molekulargewicht von 470 bis 10.000.000 hat.

12. Verwendung gemäß irgendeinem der Ansprüche 9 bis 11, worin die Verbindung ein Antikörper ist, der mit dem Polyethylenglycol, das 13C in einem höheren Anteil als das natürliche Vorkommen enthält, markiert ist.

## Revendications

1. Procédé d'imagerie par résonance magnétique comprenant l'application, à un sujet à qui on a administré un agent de contraste de résonance magnétique comprenant un poly(éthylène glycol) contenant 13C dans une proportion supérieure à l'abondance naturelle, ou un composé marqué avec ledit poly(éthylène glycol), d'impulsions d'un champ magnétique d'excitation avec un temps de répétition de 60 secondes ou moins, en acquérant ainsi des signaux de résonance magnétique en continu afin d'obtenir une image.

2. Procédé d'imagerie par résonance magnétique selon la revendication 1, dans lequel la proportion de 13C dans le poly(éthylène glycol) représente 20 à 100 % des atomes de carbone totaux.

3. Procédé d'imagerie par résonance magnétique selon la revendication 1 ou la revendication 2, dans lequel le poly(éthylène glycol) a une masse moléculaire moyenne en masse de 470 à 10 000 000.

4. Procédé d'imagerie par résonance magnétique selon l'une quelconque des revendications 1 à 3, dans lequel le composé est un anticorps marqué par le poly(éthylène glycol) contenant 13C dans une proportion supérieure à l'abondance naturelle.

5. Procédé d'acquisition de signaux de résonance magnétique, comprenant l'application, à un sujet à qui on a administré un agent de contraste de résonance magnétique comprenant un poly(éthylène glycol) contenant 13C dans une proportion supérieure à l'abondance naturelle, ou un composé marqué avec ledit poly(éthylène glycol), d'impulsions d'un champ magnétique d'excitation avec un temps de répétition de 60 secondes ou moins, en acquérant ainsi des signaux de résonance magnétique en continu.

6. Procédé d'imagerie par résonance magnétique selon la revendication 5, dans lequel la proportion de 13C dans le poly(éthylène glycol) représente 20 à 100 % des atomes de carbone totaux.

7. Procédé d'imagerie par résonance magnétique selon la revendication 5 ou la revendication 6, dans lequel le poly(éthylène glycol) a une masse moléculaire moyenne en masse de 470 à 10 000 000.

8. Procédé d'imagerie par résonance magnétique selon l'une quelconque des revendications 5 à 7, dans lequel le composé est un anticorps marqué par le poly(éthylène glycol) contenant 13C dans une proportion supérieure à l'abondance naturelle.

9. Utilisation d'un poly(éthylène glycol) contenant 13C dans une proportion supérieure à l'abondance naturelle, ou un composé marqué avec ledit poly(éthylène glycol), dans un procédé d'acquisition de signaux de résonance magnétique en continu afin d'obtenir une image, dans laquelle des impulsions d'un champ magnétique d'excitation sont appliquées avec un temps de répétition de 60 secondes ou moins.

10. Utilisation selon la revendication 9, dans laquelle la proportion de 13C dans le poly(éthylène glycol) représente 20 à 100 % des atomes de carbone totaux.

11. Utilisation selon la revendication 9 ou la revendication 10, dans laquelle le poly(éthylène glycol) a une masse moléculaire moyenne en masse de 470 à 10 000 000.

12. Utilisation selon l'une quelconque des revendications 9 à 11, dans laquelle le composé est un anticorps marqué par le poly(éthylène glycol) contenant 13C dans une proportion supérieure à l'abondance naturelle.
